(19) Europäisches Patentamt
European Patent Office
Office européen des brevets

(11) Publication number: 0 284 337 A1

(12) **EUROPEAN PATENT APPLICATION**

(21) Application number: 88302484.6

(22) Date of filing: 22.03.88

(51) Int. Cl.$^4$: C 07 D 403/14
A 61 K 31/495

(30) Priority: 25.03.87 US 30670

(43) Date of publication of application: 28.09.88 Bulletin 88/39

(84) Designated Contracting States: DE ES FR GB IT

(71) Applicant: HARBOR BRANCH OCEANOGRAPHIC INSTITUTE INC.
5600 Old Dixie Highway
Fort Pierce, FL 33450 (US)

(72) Inventor: Komoto, Shigeo
1056 24th St., S.W. Vero Beach
Florida 32962 (US)

McConnell, Oliver J.
Vero Beach
Florida 32962 (US)

(74) Representative: Marlow, Nicholas Simon et al
Reddie & Grose 16, Theobalds Road
London WC1X 8PL (GB)

The application is published incomplete as filed (Article 93 (2) EPC). The point in the description or the claim(s) at which the omission obviously occurs has been left blank.

(54) **New indole alkaloid compositions and their methods of use.**

(57) This invention relates to novel compositions which are useful as antitumor compositions, a process of producing the compositions and a method for inhibiting tumors, utilizing the compositions. More particularly, the novel compositions are indole alkaloid compositions which are derived from marine sponges of the family Biemnidae, and have the general formula:

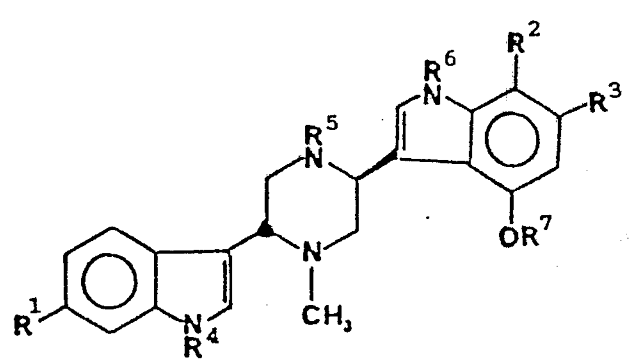

EP 0 284 337 A1

**Description**

NEW INDOLE ALKALOID COMPOSITIONS AND THEIR METHODS OF USE

This invention relates to a new organic compositions which have useful antitumor activity.

Various tumor related diseases inflict man. Considerable research has been devoted to oncology and antitumor measures. Tumors are common in a variety of mammals and the prevention, control of the growth and regression of tumors in mammals is important to man. The term tumor refers to abnormal masses of new tissue growth which is discordant with the economy of the tissue of original of the host's body as a whole.

Tumors inflict mammals and man with a variety of disorders and conditions including various forms of cancer and resultant cancerous cachexia. Cancerous cachexia refers to the symptomatic discomfort that accompanies the infliction of a mammal with a tumor. These symptoms include weakened condition of the inflicted mammal as evidenced by, for example, weight loss. The seriousness of cancer is well know, e.g., cancer is second only to heart and vascular diseases as a cause of death in man.

Considerable research and resources have been devoted to oncology and antitumor measures including chemotherapy. While various antitumor agents and methods have been developed which aid in inhibiting tumors additional methods and chemical agents are needed.

A potential source for antitumor compositions is marine plant and animal life and of particular interest are marine sponges. It has now been found that an organic composition derived from extracts of a sponge of the family Biemnidae possess useful antitumor activity.

of the formula:

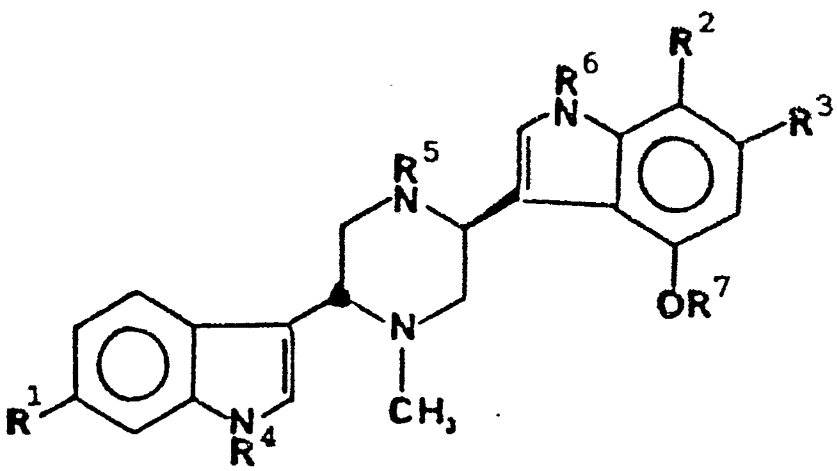

wherein $R^{1-3}$ are the same or different and are a hydrogen or halogen and $R^{4-7}$ are the same or different and are a hydrogen, lower alkyl or lower acyloxy group. In preferred embodiments $R^{1-3}$ are a hydrogen or bromine and $R^{4-7}$ are a hydrogen, methyl or acetyloxy group.

In a further preferred embodiment the composition has the formula:

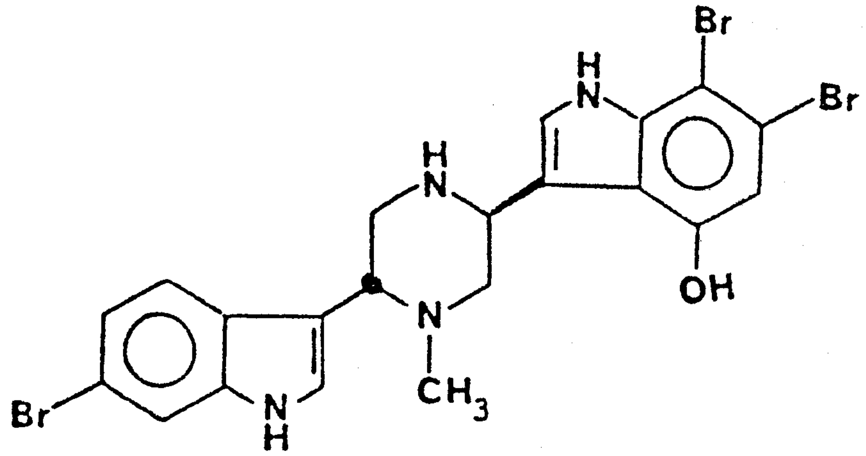

In preferred embodiments of the invention, the composition is substantially pure.

As embodied and fully described herein, the invention also comprises antitumor compositions comprising, as active ingredient, an effective antitumor amount, respectively, of the compositions of the invention and non-toxic pharmaceutically acceptable carrier or diluent.

As embodied and fully described herein, the invention also comprises a process to produce the compositions of the invention. The process comprises the steps of collection marine sponge of the family Biemnidae contacting the marine sponge with a suitable organic solvent system to obtain an extract; fractionating the extract; and isolating indole alkaloid compositions of the invention from the fractionated extract.

As embodied and fully described herein, the invention further comprises a method for inhibiting tumors comprising contacting tumor cells with an effective antitumor amount of the composition of the invention.

It is to be understood that both the foregoing general and the following detailed description are exemplary and explanatory only and are not intended to be restrictive of the invention as claimed.

## DETAILED DESCRIPTION OF THE PREFERRED EMBODIMENTS OF THE INVENTION

Reference will now be made in detail to present preferred embodiments of the invention, an example of which is illustrated in the following example section.

In accordance with the invention novel compositions are provided to achieve the objects in accordance with the purposes of the invention, as embodied and fully described herein, the invention comprises indole alkaloid compositions of the general formula I:

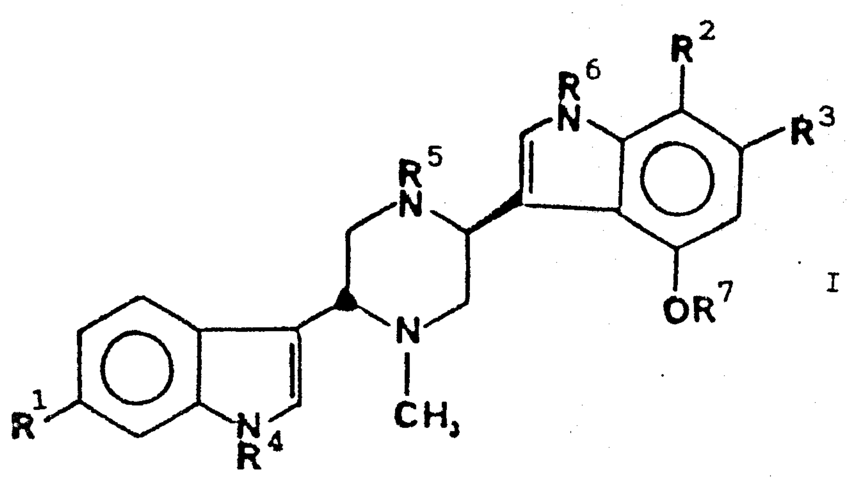

I

wherein $R^{1-3}$ are the same or different and are a hydrogen and halogen and $R^{4-7}$ are the same or different and are a hydrogen, lower alkyl or lower acetyloxy group. In preferred embodiments $R^{1-3}$ are hydrogen or bromine and $R^{4-7}$ are a hydrogen, methyl or acetyloxy group.

In a further preferred embodiment of the invention the composition has the formula, shown below, and is named biemnidin:

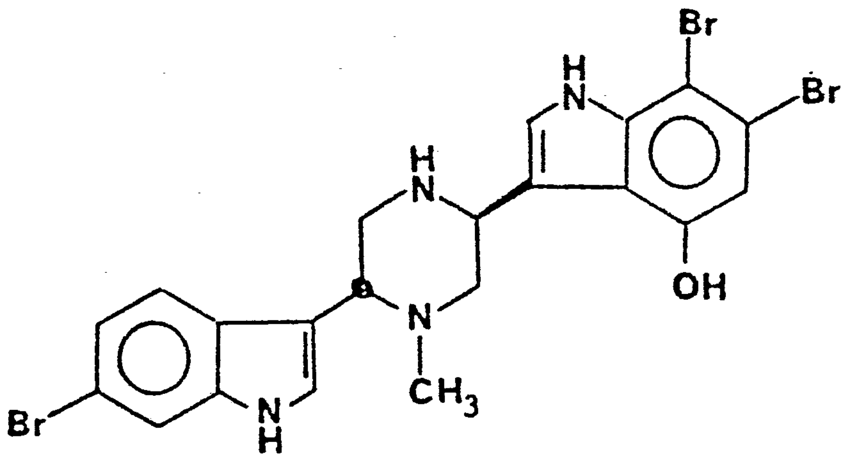

In preferred embodiments of the invention, the composition is substantially pure.

In accordance with the invention, an antitumor composition is provided comprising as active ingredient an effective antitumor amount of the composition of the invention and a non-toxic pharmaceutically acceptable carrier or diluent. While effective amounts may vary, as conditions in which an antitumor composition is used vary, a minimal dosage required for in vitro activity is generally between 0.01 and 100 micrograms per milliliter against $10^5$ tumor cells. Useful examples of non-toxic pharmaceutically acceptable carriers or diluents include, but are not limited to, the following: ethanol, dimethyl sulfoxide, methanol and glycerol.

In accordance with the invention, a method for inhibiting tumors in a host is provided comprising contacting a tumor with an antitumor amount of the composition of the invention. The composition of the invention is active for inhibiting a diverse range of tumors including, but not limited to P388 mouse leukemia cells, human lung, colon and mammary tumors such as lung carcinoma A-549, ileocecal adenocarcinoma HCT-8, and human breast adenocarcinoma cells MD-AMB-231. The effectiveness of the composition of the invention for inhibiting tumor cells and tumors indicates its usefulness for controlling tumors in hosts including mammals and for treating cancerous cachexia.

In accordance with the invention, a process is provided to produce the compositions of the invention. The process comprises the steps of collecting samples of marine sponge of the family Biemnidae; contacting the marine sponge with a suitable organic solvent system to obtain an extract; partitioning said extract by chromatography to obtain a number of fractions; and isolating the composition of the invention from the fractionated extract.

In preferred embodiments of the invention the suitable organic solvent system is selected from the group of solvents consisting of methanol, toluene, heptane, hexanes, isooctane, acetone, ethyl acetate, benzene, diethyl ether, t-butyl-methyl ether, ethanol, isopropanol, chloroform, 1,2-dichloroethane, dichloromethane, and mixtures thereof. A particularly preferred extracting solvent is a mixture of methanol and toluene.

While those solvents listed above are the presently preferred choices for the solvents useful in accordance with the invention, other suitable solvents may be substituted. A suitable solvent system should be capable of extracting the composition of the invention from other components of the sponge. Different ratios of solvents and any combination may be used in the solvent system of the invention as would be known to those skilled in the art.

Compositions according to the invention are synthesized and/or isolated by various fractionation, and chromatographic techniques from the extracts obtained. Any suitable fractionation and isolation technique, as known to those skilled in the art, may be utilized in accordance with the process of the invention. Preferred isolation techniques include various chromotography techniques such as vacuum liquid chromotography with suitable columns as would be known to those skilled in the art (e.g., Kiesel gel 60-H) eluted with a suitable solvent such as, for example, methylene chloride, isopropanol, heptane, methanol, ethanol, dichloromethane, ethyl acetate, hexanes, isooctane, dichloromethane, chloroform, 1,2-dichloroethane, benzene, toluene, t-butyl-methyl ether, diethyl ether, acetone, and mixtures thereof. Particularly preferred eluents are methylene chloride and isopropanol and mixtures thereof.

A more detailed description and explanation of a preferred embodiment of the process of the invention to produce the composition of the invention is provided in the examples section.

It is therefore apparent that the composition of the invention, the process for producing the composition of the invention and the methods for utilizing the composition of the invention to inhibit tumors, viruses and fungus growth fulfill the objects of the invention.

EXAMPLE

The invention will now be illustrated by example. The example is not intended to be limiting of the scope of the present invention. In conjunction with the detailed and general description above, the example provides

further understanding of the present invention and outlines a process for producing the compositions of the invention.

The following example represents a preferred embodiment of the compositions, processes and methods of the invention for satisfying the stated objects of the invention. The starting materials and reagents in the example whose methods of preparation are not indicated are commercially available from sources known to the art as chemical supply houses.

EXAMPLE 1

Preparation of biemnidin

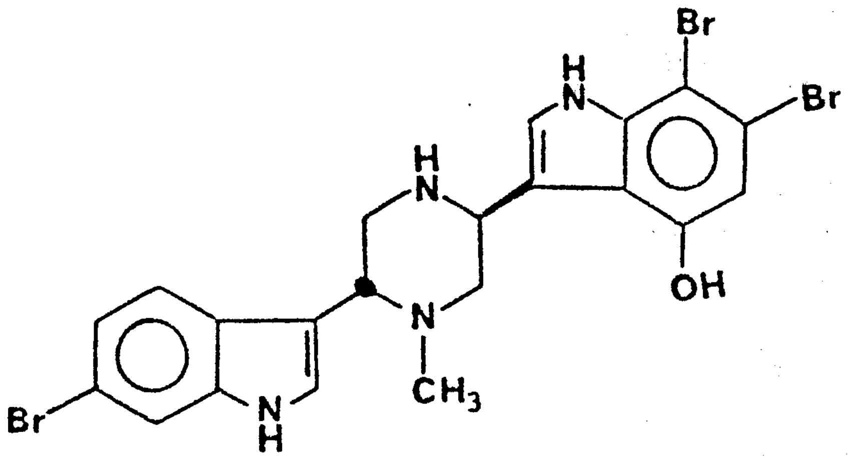

1

The marine sponge family Biemnidae was collected at a depth of 480 feet in Sweeting, Bahamas. An extract of the sponge was prepared by homogenizing the frozen organism and repeatedly steeping with methanol/toluene (3:1). 0.5gms. of crude extract was chromatographed by vacuum liquid chromatography on Kiesel gel 60 (EM Science) using $CHCl_3$/isopropanol as the eluent. 90 milligrams of pure biemnidin (1) was eluted in fraction 2.

| Fraction | Eluent | Weight (mg) |
|---|---|---|
| 1 | $CHCl_3$/i-PrOH=5:1 | 230 |
| 2 | $CHCl_3$/i-PrOH=1:1 | 90 |

Biemidin has structure 1. It is a slightly white brown powder. The physical and spectral characteristics of 1 are listed below. $[\alpha]^{20}_{D}$ - 3° (C=13.2, acetone)

IR: KBr; $cm^{-1}$ 3420, 3380, 3010, 2950, 2840, 2790, 1700, 1610, 1540, 1470, 1450, 1400, 1330, 1280, 1210.

UV: MeOH; $\lambda$max(nm) 220 ($\varepsilon$= 52600), 275 ($\varepsilon$= 11700), 286 sh, ($\varepsilon$= 10900), 293, sh, ($\varepsilon$= 10100).

MS: HRFAB; 580.9173 for $C_{21}H_{20}Br_3N_4O$ ($M^+$ + H) ($\Delta$ 2.4)

NMR: 360MHz in acetone-$d_6$

Proton: $\delta$10.8 (br, s, 1H), 10.5 (br, s, 1H), 7.84 (d, J=8.6, 1H), 7.59 (d, J=1.8, 1H) 7.36 (d, J=1.8, 1H), 7.27 (S, 1H), 7.13 (dd, J=1.8 and 8.6, 1H), 6.70 (S, 1H), 4.35 (dd, J=2.3 and 10.3, 1H), 3.46 (dd, J=3.9 and 11.3, 1H), 3.33 (dd, J=11.3 and 11.9, 1H), 3.13 (dd, J=2.3 and 11.9, 1H), 3.05 (br, 1H), 2.39 (dd, J=10.3 and 11.0, 1H), 2.04 (s, 3H).

Carbon: $\delta$153.1(s), 138.7(s), 138.1(s), 126.2(s), 125.3(d), 122.6(d), 122.4(d), 122.2(d), 118.5(s), 118.2(s), 117.7(s), 115.7(s), 115.4(s), 115.2(d), 110.5(d), 95.6(s), 63.4(t), 61.8(d), 53.4(d), 52.4(t), 43.8(q).

ANTITUMOR ACTIVITIES OF THE COMPOSITIONS OF THE INVENTION

The following assay method was utilized to illustrate the antitumor effectiveness of the indole alkaloid compositions of the invention.

P388 MOUSE LEUKEMIA CELL ASSAY

Maintenance of Cell Line

P388 mouse leukemia cells are grown in Dulbecco MEM medium with 10% horse serum, 4mM glutamine, and 20ug/ml gentamycin (Biologos, Inc.). Cells are incubated in 10% $CO_2$ and subcultured 2 times per week.

PROCEDURE

1. Add composition to each well of a 24-well plate or tube and allow solvent to evaporate to dryness.
2. Add 2ml ($1.2 \times 10^5$) cells to each well or tube and mix.
3. Incubate in 10% $CO_2$ at 37° for 48 hours.
4. Read plates with an inverted microscope, scoring activity from 1+ to 4+ as follows: ND (not detectable), >90%; 1+, 75-90%; 2+, 50-74%; 3+, 25-49%; 4+, <25% of control growth. Alternatively, the activity may be designated as $IC_{50}$ concentration which is the concentration of composition required to inhibit 50% of cell growth on the plate.

Cell counts are performed on each tube and results are reported as percent of control.

HUMAN TUMOR CELL LINE ASSAY

Maintenance of Cell Line

HCT-8 human colon tumor cells are grown in RPM1 1640 medium (GIBCO). A-549 human lung carcinoma cells and MD-AMB-231 human breast cancer cells are cultured in Dulbecco medium (Biologos, Inc.). All media are supplemented with 10% fetal bovine serum and contain 50 ug/ml gentamycin. All human tumor cell lines are incubated at 5% $CO_2$ at 37° and subcultured once a week. The seeding levels are 350,000 MCF-7 cells, 60,000 HCT-8 cells and 300,000 A-549 cells per T-25 Flask. Vinblastine is used as a positive control.

PROCEDURE

1. Seed 1ml cell (5000 HCT-8 , 8000 A-549, 12000 MD-AMB-231) in each well of a 24-well plate.
2. Incubate in a $CO_2$-incubator for 48 hours.
3. Add composition to each well and incubate for an additional 120 hours.
4. Discard medium and stain with methylene blue (HCT-8) or crystal violet (A-549 and MD-AMB-231).
5. Compare cell density of drug-treated well with that of the control (no drug added) as follows: ND (not detectable), >90%; 1+, 75-90%; 2+, 50-74%; 3+, 25-49%, 4+, <25% of control growth.

Positive control Vinblastine in aqueous solution at the following concentrations.

| Solution Conc. | Amt added | Final conc. in test |
|---|---|---|
| 5 mg/ml | 2 µl | 5 µg/ml |
| 1 mg/ml | 2 µl | 1 µg/ml |
| 0.1 mg/ml | 2 µl | 0.1 µg/ml |
| 0.05 mg/ml | 2 µl | 0.05 µg/ml |

The results of the above assays are summarized in Table 1.

Table 1

Antitumor Activity of Biemnidin (1)

Mouse: P388: $IC_{50}$ = 15 µg/ml

| | | | Activity at prescribed doses (µg/ml) | | |
|---|---|---|---|---|---|
| Human: | Tumor | Cell Line | 50 | 10 | 1 |
| | Colon | HCT-8 | 4+ | 4+ | ND |
| | Lung | A-549 | 4+ | 4+ | ND |
| | Mammary | MD-AMB-231 | 4+ | 4+ | ND |

The above data reports the in vitro activity of the compositions of the invention for inhibiting tumors. The above results indicate, as would be known to those skilled in the art, that the composition biemnidin and the other indole alkaloid compositions of the invention are useful for inhibiting tumors in vivo in hosts, including mammals, and for treating diseases caused thereby. Derivatives of the compositions of the invention such as choride derivatives may be prepared which may have antitumor activities. The composition described herein may have other useful applications such as, for example, analgesic applications or as starting materials for the preparations of other compositions. Therapeutic application of the compositions of the present invention can be accomplished by any suitable therapeutic method and technique as is presently or prospectively known to those skilled in the art. Further, the composition of the invention may have use as a starting material or intermediate for the preparation of other useful compositions.

**Claims**

1. A composition according to the formula:

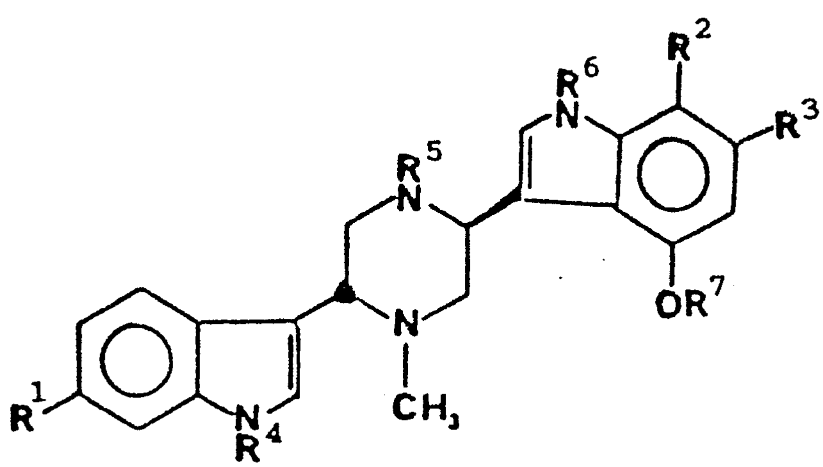

wherein $R^{1-3}$ are the same or different and are a hydrogen or halogen, and $R^{4-7}$ are the same or different and are a hydrogen, lower alkyl or lower acyloxy group.

2. A composition according to claim 1 wherein $R^{1-3}$ are hydrogen or bromine and $R^{4-7}$ are a hydrogen, methyl, or acetyloxy group.

3. A composition of the formula:

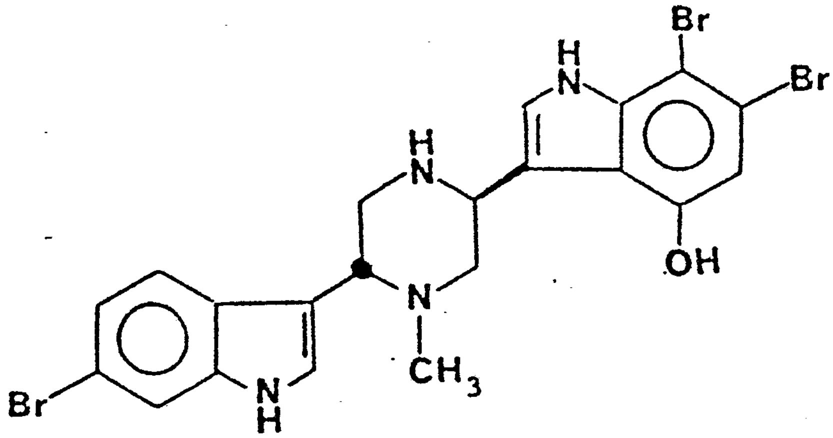

4. The composition according to any preceding claim, the composition being substantially pure.

5. An antitumor composition comprising, as active ingredient, an effective antitumor amount of the composition of any of claims 1 to 4 and a non-toxic pharmaceutically acceptable carrier or diluent.

6. A process for producing a composition according to any of claims 1 to 4 comprising:

collecting marine sponge of the family Biemnidae;

contacting the sponge with a suitable organic solvent system; obtaining a solvent extract from the sponge;

fractionating the extract; and

isolating a composition according to any of claims 1 to 4 from the fractionated extract.

7. A process to claim 6 in which the solvent system is methanol, toluene, heptane, hexanes, isooctane, acetone, ethanol, isopropanol, chloroform, benzene, diethyl ether, t-butyl-methyl ether, 1,2-dichloroethane, dichloromethane or any combination thereof.

8. A process according to claim 6 or 7 in which the solvent extract is fractionated and isolated by vacuum liquid chromatography using as an eluent methylene chloride, chloroform, isopropanol, heptane, hexanes, isooctane, dichloromethane, 1,2-dichloroethane, benzene, toluene, ethyl acetate, t-butyl-methyl ether, diethyl ether, methanol, ethanol, acetone, or combinations thereof.

9. A process according to any of claim 6 to 8 comprising:

collecting marine sponge of the family Biemnidae;

contacting said sponge with a 3:1 mixture of methanol and toluene to obtain a solvent extract thereof;

fractionating the extract by vacuum liquid chromtography using a 5:1 mixture of chloroform and isopropanol as a first eluent to obtain a first extract and a 1:1 mixture of chloroform and isopropanol as a second eluent to obtain a second extract; and

isolating a composition according to any of claims 1 to 4 from the second extract.

10. A method for inhibiting tumors in a host comprising contacting a tumor with an effective antitumor amount of the composition of any of claims 1 to 5.

11. A therapeutic method for treating cancerous cachexia caused by the presence of a tumor in a host comprising contacting cells of said tumor with an effective antitumor amount of a composition according to any of claims 1 to 5.

12. A therapeutic method for treating cancerous cachexia caused by the presence of a tumor in a mammalian host comprising contacting cells of said tumor with an effective antitumor amount of a composition according to any of claims 1 to 5.

European Patent Office

# PARTIAL EUROPEAN SEARCH REPORT

which under Rule 45 of the European Patent Convention shall be considered, for the purposes of subsequent proceedings, as the European search report

Application number: EP 88302484.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 103, no. 1, July 8, 1985, Columbus, Ohio, USA<br>MUELLER, JOHANN; PFEUFFER, LUDWIG; PINDUR, ULF "Regioselective synthesis of symmetric 3,3'-bisindolylethenes"<br>page 557, column 1, abstract-no. 6 178f<br>& Wuerzburg, Fed. Rep. Ger. Chem.-Ztg. 1985, 109(1), 15-16<br>-- | 1 | C 07 D 403/14<br>A 61 K 31/495 |
| A | CHEMICAL ABSTRACTS, vol. 95, no. 9, August 31, 1981, Columbus, Ohio, USA<br>COLONNA, MARTINO; GRECI, LUCEDIO; POLONI, MARINO "Ipso-substitutions. Reactions of 3-substituted indoles with benzoyl peroxide"<br>page 754, column 2, abstract-no. 80 635k<br>& Tetrahedron Lett. 1981, 22(12), 1143-4<br>-- | 1 | |

TECHNICAL FIELDS SEARCHED (Int. Cl.4)

C 07 D 403/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9

Claims searched incompletely: -

Claims not searched: 10-12

Reason for the limitation of the search:

(Art. 52(4) EPC; method for treatment of the human or animal body by therapy)

| Place of search | Date of completion of the search | Examiner |
|---|---|---|
| VIENNA | 01-06-1988 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number
EP 88302484.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 85, no. 1, July 5, 1976, Columbus, Ohio, USA<br>TSUBOYAMA, SEI; TSUBOYAMA, KAORU; TANJI, SHOJI; YANAGITA, MASAYA "Syntheses of optically active 2,5-dialkylpiperazines" page 451, column 2, abstract-no. 5 592j<br>& Nippon Kagaku KAISHI 1976, (3), 540-2<br>---- | 1 | |
| | | | TECHNICAL FIELDS SEARCHED (Int. Cl.4) |